# EUROPÄISCHE PATENTANMELDUNG

(11) **EP 4 227 867 A1**
(43) Veröffentlichungstag der Anmeldung: **16.08.2023**
(21) Anmeldenummer: 23153950.3
(22) Anmeldetag: 30.01.2023
(51) Int. Cl.: G06Q 10/04, G06Q 10/20, G16H 20/40, G16H 30/40, G16H 40/40, A61B 90/70, A61B 90/00

(54) **VERFAHREN ZUR HANDHABUNG VON STATUSINFORMATIONEN MEDIZINISCHER GERÄTE, UND SYSTEM**

(30) Priorität: 15.02.2022 US 202263310173 P
(71) Anmelder: OLYMPUS WINTER & IBE GMBH, 22045 Hamburg (DE)
(72) Erfinder: Tessmann, Ralf, 22149 Hamburg (DE); Jaskola, Sascha, 22767 Hamburg (DE); Sverdlov, Juri, 22767 Hamburg (DE); Siegmund, Ralf, 22926 Ahrensburg (DE); Stefka, Veronika, 22089 Hamburg (DE); Karstens, Christian, 22046 Hamburg (DE)
(74) Vertreter: Noack, Andreas

(57) **Zusammenfassung**

Hiermit wird ein Verfahren zur Handhabung von Statusinformationen medizinischer Geräte bereitgestellt, mit den Schritten: Empfangen von Statusinformationsdaten von einem oder mehreren medizinischen Geräten, wobei die Statusinformationsdaten in einem Datenformat empfangen werden, das für jedes medizinische Gerät spezifisch ist; Bestimmen, dass die Statusinformationsdaten einen Fehlerzustand des einen oder der mehreren medizinischen Geräte anzeigen; Zuordnen einer oder mehrerer globaler Fehlerkategorien aus einer vorbestimmten Liste globaler Fehlerkategorien zu jedem Fehlerzustand, wobei die globalen Fehlerkategorien nicht spezifisch für ein bestimmtes medizinisches Gerät sind; und Ausgeben von Fehlerinformationsdaten, umfassend: Identifikationsdaten, die ein bestimmtes medizinisches Gerät identifizieren; Statusinformationsdaten, die von dem bestimmten medizinischen Gerät empfangen werden; und die eine oder mehrere globale Fehlerkategorien, die dem erkannten Fehlerstatus zugeordnet sind.

## Beschreibung

### Bereich der Erfindung

Die vorliegende Offenbarung bezieht sich auf Verfahren zur Handhabung von Statusinformationen medizinischer Geräte. Insbesondere bezieht sich die Offenbarung auf die Handhabung von Statusinformationen medizinischer oder chirurgischer Endoskope und von Endoskop-Aufbereitungsmaschinen.

### Hintergrund

Medizinische und chirurgische Endoskope werden in der modernen Medizin zur Untersuchung und Behandlung von Patienten mit einem breiten Spektrum von Erkrankungen eingesetzt. Solche Endoskope sind hochkomplizierte und teure Instrumente. Anders als viele medizinische Geräte werden Endoskope daher nicht nach einmaligem Gebrauch entsorgt, sondern nach sorgfältiger Aufbereitung viele Male wiederverwendet.

Die Aufbereitungsverfahren sind darauf ausgelegt, Schmutz, Flecken, Krankheitserreger und Rückstände von einem Endoskop vor der Wiederverwendung zu entfernen, so dass eine Kreuzkontamination von Patienten sicher verhindert werden kann. Während einige Schritte des Aufbereitungsverfahrens manuell durchgeführt werden, werden die meisten Schritte von automatischen Endoskop-Aufbereitungsmaschinen ausgeführt, wie sie von der Anmelderin unter dem Namen "ETD" angeboten werden. Je nach den spezifischen Aufbereitungsverfahren kann die Aufbereitung auch die Trocknung der Endoskope in Trockenschränken umfassen. Im Zusammenhang mit der vorliegenden Offenlegung kann der Begriff "Endoskop-Aufbereitungsmaschine" auch solche Trockenschränke umfassen.

Um sicherzustellen, dass ein chirurgisches Gerät wie ein Endoskop sicher wiederverwendet werden kann, werden die Aufbereitungsverfahren automatisch überwacht und die Ergebnisse in einer Datenbank gespeichert. Diese Ergebnisse können Daten umfassen, die für ein einzelnes Endoskop spezifisch sind, wie z. B. die Ergebnisse von Durchlässigkeitstests für Kanäle, Leckagetests für Endoskope oder ähnliches, und Daten, die für ein Aufbereitungsgerät spezifisch sind, wie z. B. Dosiermengen von Reinigungs- oder Desinfektionsmitteln, Verarbeitungstemperaturen, Dauer von Spülzyklen oder ähnliches. Liegt ein Parameter außerhalb eines bestimmten Bereichs, wird eine Fehlermeldung erzeugt, die anzeigt, dass ein Endoskop nach der Aufbereitung nicht einsatzbereit ist und/oder dass ein Aufbereitungsgerät gewartet werden muss. Solche Fehlermeldungen enthalten in der Regel einen alphanumerischen Fehlercode, der für den Typ des Aufbereitungsgeräts, bei dem der Fehler aufgetreten ist, spezifisch ist, manchmal zusammen mit einer kurzen Klartextbeschreibung.

In vielen Krankenhäusern und größeren Endoskopiepraxen ist eine Vielzahl von Aufbereitungsmaschinen installiert, darunter manchmal auch Aufbereitungsmaschinen verschiedener Typen. Die Statusinformationen dieser Maschinen werden in einem zentralen Kontrollsystem gesammelt und dem Benutzer über eine Benutzeroberfläche angezeigt.

Für einen Benutzer kann es schwierig sein, die Art und den Schweregrad einer Fehlermeldung zu verstehen, da es verschiedene Arten von Aufbereitungsgeräten gibt und die verschiedenen Aufbereitungsgeräte unterschiedliche Fehlercodes liefern.

Es wäre von Vorteil, die Handhabung der Statusinformationen medizinischer Geräte zu verbessern, damit ein Benutzer die Art eines Fehlerzustands leichter verstehen und entsprechende Gegenmaßnahmen einleiten kann.

Darüber hinaus wäre es von Vorteil, die Handhabung von Statusinformationen medizinischer Geräte zu verbessern, damit anstehende Probleme eines Geräts erkannt werden können, bevor das Gerät in einen Fehlerzustand gerät.

Es wäre auch von Vorteil, die Handhabung von Statusinformationen medizinischer Geräte zu verbessern, damit leichter festgestellt werden kann, ob ein Fehler auf eine Fehlfunktion der Maschine oder auf menschliches Versagen zurückzuführen ist.

### Zusammenfassung der Erfindung

Die vorliegende Offenbarung stellt ein Verfahren zur Behandlung von Statusinformationen medizinischer Geräte bereit, das die folgenden Schritte umfasst: Empfangen von Statusinformationsdaten von einem oder mehreren medizinischen Geräten, wobei die Statusinformationsdaten in einem für jedes medizinische Gerät spezifischen Datenformat empfangen werden; Feststellen, dass die Statusinformationsdaten einen Fehlerzustand des einen oder der mehreren medizinischen Geräte anzeigen; Bestimmen, dass die Statusinformationsdaten einen Fehlerzustand des einen oder der mehreren medizinischen Geräte anzeigen; Zuordnen einer oder mehrerer globaler Fehlerkategorien aus einer vorbestimmten Liste globaler Fehlerkategorien zu jedem Fehlerzustand, wobei die globalen Fehlerkategorien nicht spezifisch für ein bestimmtes medizinisches Gerät sind; und Ausgeben von Fehlerinformationsdaten, die Identifikationsdaten, die ein bestimmtes medizinisches Gerät identifizieren, Statusinformationsdaten, die von dem bestimmten medizinischen Gerät empfangen wurden, und die eine oder mehrere Fehlerkategorien umfassen, die dem erfassten Fehlerzustand zugeordnet sind.

Durch die Ausgabe der einen oder mehreren globalen Fehlerkategorien im Falle eines Fehlerzustands eines medizinischen Geräts ist es für einen Benutzer oder Bediener einfacher, die Art des Fehlers zu verstehen, der bei dem Gerät aufgetreten ist.

Die Liste der Fehlerkategorien kann Fehlerkategorien enthalten, die unterschiedliche Schweregrade der jeweiligen Fehlerzustände anzeigen. Die Liste der Fehlerkategorien kann eine oder mehrere Fehlerkategorien enthalten: eine erste Fehlerkategorie, die einen Fehler mit hohem Schweregrad anzeigt; eine zweite Fehlerkategorie, die einen Fehler mit mittlerem Schweregrad anzeigt; und eine dritte Fehlerkategorie, die einen Fehler mit niedrigem Schweregrad anzeigt. Fehler mit hohem Schweregrad können auch als "rote Fehler" bezeichnet werden und können ein Versagen eines medizinischen Geräts, das die Aufmerksamkeit eines Bedieners erfordert, ein Versagen eines Aufbereitungsverfahrens, das dazu führt, dass ein oder mehrere Endoskope nach dem Verfahren nicht sicher verwendet werden können, oder Ähnliches umfassen. Zu den Fehlern mittleren Schweregrads, die auch als "gelbe Fehler" bezeichnet werden können, gehört der Zustand eines medizinischen Geräts oder eines Prozessparameters oder das Ergebnis eines Verfahrens, das außerhalb eines optimalen Bereichs, aber innerhalb der zulässigen Grenzwerte liegt. Zu den Fehlern mit geringem Schweregrad gehören Vorabmeldungen über Gerätekomponenten, die sich dem Ende ihrer geplanten Nutzungsdauer nähern, über Prozesschemikalien, die sich dem Ende ihrer Haltbarkeitsdauer nähern, oder über andere anstehende Wartungsaktivitäten.

Die Liste der Fehlerkategorien kann außerdem Fehlerkategorien enthalten, die anzeigen, ob der Fehlerzustand durch menschliches Versagen verursacht wurde oder nicht.

Die Fehlerinformationsdaten können Daten enthalten, die einen Bediener auffordern, eine Fehlerbehandlungsprozedur durchzuführen, wobei die Fehlerbehandlungsroutine von der einen oder mehreren Fehlerkategorien abhängt, die dem Fehlerzustand zugeordnet sind. Die Fehlerinformationen können ferner Informationen zur Fehlerbehebung enthalten, die für das von dem Fehlerzustand betroffene medizinische Gerät spezifisch sind.

Das Verfahren kann ferner den wiederholten Empfang von Statusinformationsdaten von dem einen oder den mehreren medizinischen Geräten umfassen und die Ausgabe der Fehlerinformationsdaten beenden, wenn die Statusinformation anzeigt, dass der Fehlerzustand behoben wurde.

Das Verfahren kann ferner die Bereitstellung statistischer Informationen umfassen, die eines oder mehrere der folgenden Elemente umfassen:
Eine Anzahl oder Häufigkeit bestimmter Fehlerzustände oder Fehler einer bestimmten Fehlerkategorie innerhalb eines vorgegebenen oder wählbaren Zeitraums, eine durchschnittliche oder kumulierte Ausfallzeit, die durch bestimmte Fehlerzustände oder Fehler einer bestimmten Fehlerkategorie verursacht wird, oder eine Rangliste der häufigsten Fehlerzustände oder Fehlerkategorien.

Die vorliegende Offenbarung stellt ferner ein Verfahren zur Handhabung von Statusinformationen medizinischer Geräte bereit, mit den Schritten: wiederholtes Empfangen von Statusinformationsdaten von einem oder mehreren medizinischen Geräten, Speichern der empfangenen Statusinformationsdaten in einer Datenbank, Bestimmen, ob die Statusinformationsdaten einen Fehlerzustand eines ersten des einen oder der mehreren medizinischen Geräte anzeigen; und nach dem Bestimmen, dass die Statusinformationsdaten einen Fehlerzustand des ersten des einen oder der mehreren medizinischen Geräte anzeigen: Analysieren der gespeicherten Statusinformationsdaten nach ersten Datenmustern, die den Fehlerzustand vorhersagen; Durchsuchen gespeicherter Statusinformationsdaten, die von mindestens einem zweiten des einen oder der mehreren medizinischen Geräte, das nicht das erste ist, empfangen wurden, nach zweiten Datenmustern, die den ersten Datenmustern ähnlich sind; Bestimmen einer Wahrscheinlichkeit, dass das zweite des einen oder der mehreren medizinischen Geräte innerhalb eines gegebenen Zeitraums auf einen Fehlerzustand trifft, auf der Grundlage einer Ähnlichkeit zwischen den zweiten Datenmustern und den ersten Datenmustern; und wenn die Wahrscheinlichkeit einen Schwellenwert überschreitet, Ausgeben von vorhergesagten Fehlerinformationsdaten, die Identifikationsdaten, die das zweite medizinische Gerät identifizieren, Statusinformationsdaten, die von dem zweiten medizinischen Gerät empfangen werden, und vorhergesagte Fehleridentifikationsdaten, die den vorhergesagten Fehlerzustand identifizieren, umfassen. Durch die Ausgabe von Informationsdaten über vorhergesagte Fehler ist es für einen Benutzer möglich, Gegenmaßnahmen einzuleiten, die das Auftreten des vorhergesagten Fehlers verhindern können.

Das Verfahren kann ferner umfassen, dass jedem vorhergesagten Fehlerzustand mindestens eine vorhergesagte Fehlerkategorie aus einer vorbestimmten Liste vorhergesagter Fehlerkategorien zugewiesen wird; und dass die zugewiesene vorhergesagte Fehlerkategorie als Teil der vorhergesagten Fehleridentifikationsdaten ausgegeben wird. Die Liste der vorhergesagten Fehlerkategorien kann vorhergesagte Fehlerkategorien umfassen, die unterschiedliche Schweregrade der jeweiligen vorhergesagten Fehlerzustände anzeigen. Die Liste der vorhergesagten Fehlerkategorien kann mindestens eine vorhergesagte Fehlerkategorie umfassen, die anzeigt, dass der vorhergesagte Fehlerzustand unbekannt ist. Der Begriff "unbekannt" umfasst hier insbesondere alle Fehlerzustände, die von einem Hersteller des betroffenen medizinischen Geräts nicht vollständig analysiert wurden.

Mindestens einer der Schritte Analyse, Suche und Bestimmung kann einen oder mehrere Mustererkennungsalgorithmen verwenden. Mindestens einer der Analyseschritte, der Suchschritt und der Bestimmungsschritt können mit einem künstlichen neuronalen Netz oder einer Support-Vector-Maschine durchgeführt werden.

Die vorliegende Offenbarung stellt ferner ein System bereit, das mindestens eine Endoskop-Aufbereitungsmaschine, eine Datenbank und eine Steuereinheit umfasst, wobei die Steuereinheit so eingerichtet ist, dass sie ein Verfahren wie oben beschrieben durchführt.

### Kurze Beschreibung der Zeichnungen

Im Folgenden werden einige beispielhafte Ausführungsformen der vorliegenden Offenbarung anhand von Zeichnungen erläutert. Die Zeichnungen zeigen:
Abb. 1: ein Endoskop,
Abb. 2: ein Endoskop-Aufbereitungsgerät,
Abb. 3: Ein System zur Handhabung von Statusdaten,
Abb. 4: Schematische Darstellung eines computergestützten Fehlerklassifizierungssystems (ECS).

### Detaillierte Beschreibung der Zeichnungen

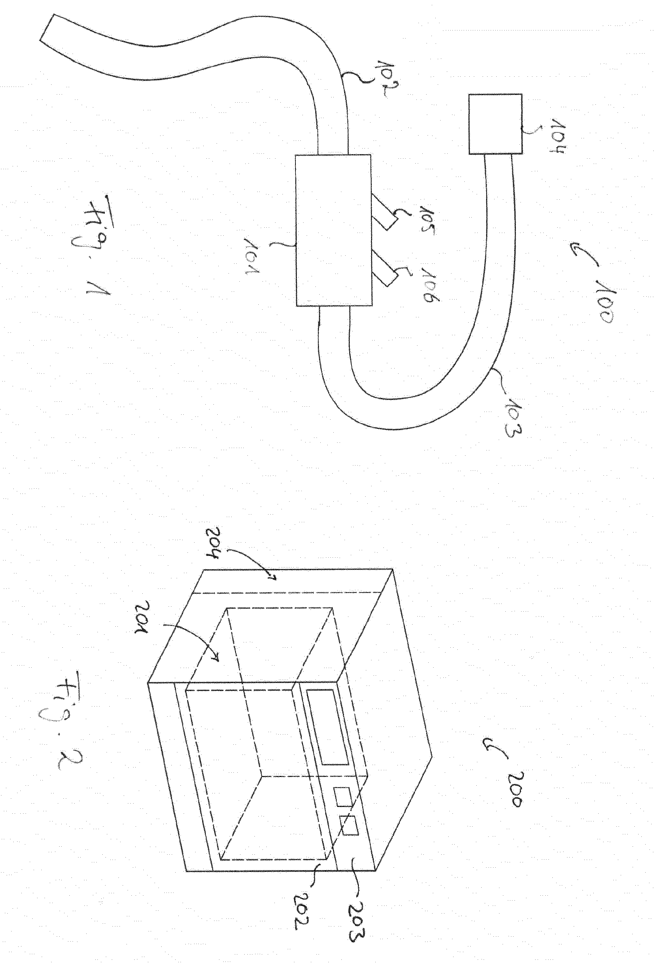

Fig. 1 zeigt ein Endoskop 100. Das Endoskop 100 umfasst einen Hauptkörper 101, einen flexiblen Schaft 102, ein Versorgungskabel 103 und einen Anschlussstecker 104. Am Hauptkörper 101 sind zwei Behandlungskanalöffnungen 105, 106 vorgesehen. Die Behandlungskanalöffnungen 105, 106 bieten Zugang zu Kanälen (nicht dargestellt) im flexiblen Schaft 102, die in einem distalen Abschnitt des flexiblen Schafts 102 enden. Die Kanäle ermöglichen das Einführen von chirurgischen Instrumenten durch den flexiblen Schaft 102 in Richtung einer Operationsstelle sowie die Injektion oder Aspiration von Flüssigkeiten.

Über das Versorgungskabel 103 und den Anschlussstecker 104 kann das Endoskop 100 mit einer Kamerasteuerung (nicht dargestellt) verbunden werden, die eine Videokamera (nicht dargestellt) im distalen Ende des flexiblen Schafts 102 steuert. Die Kamerasteuerung kann auch eine Lichtquelle zur Einkopplung von Beleuchtungslicht in das Endoskop 100

umfassen, das am distalen Ende des Schafts 102 zur Beleuchtung eines zu untersuchenden oder zu behandelnden Bereichs ausgestrahlt wird.

Ähnliche Endoskope sind z.B. aus der WO21/199180A1 bekannt.

Nach jedem Gebrauch müssen Endoskope wie das Endoskop 100 sorgfältig aufbereitet werden, bevor sie bei einem anderen Patienten eingesetzt werden können. Die Aufbereitung umfasst die mechanische und chemische Reinigung der äußeren Oberfläche des Endoskops 100 sowie aller internen Behandlungskanäle. Die chemische Reinigung der Endoskope erfolgt in hochentwickelten Endoskop-Aufbereitungsmaschinen.

Abb. 2 zeigt ein Beispiel für eine Endoskop-Aufbereitungsmaschine 200. Die Endoskop-Aufbereitungsmaschine 200 umfasst eine interne Aufbereitungskammer 201, die durch eine Fronttür 201 zum Be- und Entladen von Endoskopen zugänglich ist (in Fig. 2 nicht dargestellt). An der Vorderseite des Endoskop-Aufbereitungsgeräts 200 befinden sich Bedienelemente 203. Die Bedienelemente 203 können ein Display und eine oder mehrere Tasten zur Bedienung des Endoskop-Aufbereitungsgeräts 200 umfassen. An der Rückseite des Endoskop-Aufbereitungsgeräts 200 befindet sich ein Staufach 204 zur Aufbewahrung von Tanks mit den erforderlichen Chemikalien wie Reinigungs- und Desinfektionsmitteln. Anschlüsse für Frisch- und Abwasser, elektrische Versorgung und Datenkommunikation sind nicht dargestellt.

Ähnliche Endoskop-Aufbereitungsmaschinen sind bei der Anmelderin unter dem Namen "ETD" erhältlich. Die Endoskop-Aufbereitungsmaschine 200 ist nur ein mögliches Beispiel für Endoskop-Aufbereitungsmaschinen. Andere verfügbare Endoskop-Aufbereitungsmaschinen umfassen Top-Loading-Maschinen und Maschinen mit einem "Clean-Side/Dirty-Side"-Design, bei dem gebrauchte Endoskope durch eine erste Tür auf einer ersten (schmutzigen) Seite in die Maschine geladen und nach der Verarbeitung durch eine zweite Tür auf einer zweiten (sauberen) Seite entladen werden. Im Zusammenhang mit der vorliegenden Offenbarung kann der Begriff "Aufbereitungsmaschine" auch Trockenschränke umfassen, die zum Trocknen der Kanäle von Endoskopen nach der Aufbereitung verwendet werden. Solche Trockenschränke sind bei der Anmelderin unter dem Namen "EDC Plus" erhältlich.

Die Endoskop-Aufbereitungsmaschine 200 liefert über eine Datenverbindung regelmäßig Statusdaten über die Maschine selbst und Statusdaten über die aufzubereitenden Endoskope. Die Statusdaten werden von einem Steuerungssystem empfangen.

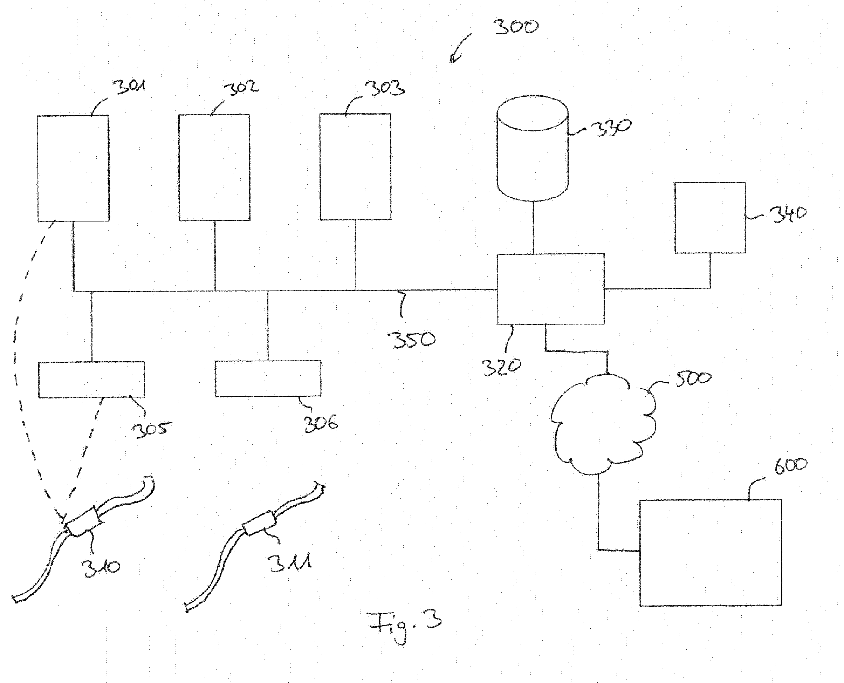

Abb. 3 zeigt ein System 300 zur Verwaltung von Statusdaten für mehrere medizinische Geräte. Zu den medizinischen Geräten gehören eine Reihe von Endoskop-Aufbereitungsmaschinen 301, 302, 303, Kamerasteuerungen 305, 306 und Endoskopen 310, 311.

Das System 300 umfasst ferner eine Steuereinheit 320, eine Datenbank 330 und eine Benutzeroberfläche 340. Die Steuereinheit 320 ist über ein kabelgebundenes oder drahtloses Netzwerk 350, z. B. ein Krankenhausnetzwerk, mit den Endoskop-Aufbereitungsmaschinen 301, 302, 303 und den Kamerasteuerungen 305, 306 verbunden.

Obwohl nicht im Detail dargestellt, kann die Steuereinheit 320 Standard-Computerhardware wie einen Prozessor, Speicher und Bussysteme zur Verbindung des Prozessors mit dem Speicher und anderen Komponenten verwenden. Derartige Standard-Computerhardware ist dem Fachmann bekannt. Im Speicher, der aus einem oder mehreren der folgenden Elemente bestehen kann: Random-Access-Memory (RAM), Read-Only-Memory (ROM), Flash-Memory und Hard-Disk-Drives (HDD), können computerlesbare Befehle gespeichert werden, die den Prozessor dazu veranlassen, eines der in dieser Beschreibung beschriebenen Verfahren auszuführen.

Bei der Datenbank 330 kann es sich um eine interne Datenbank handeln, die in der Steuereinheit 320 untergebracht ist, oder um eine externe Datenbank, die auf einem von der Steuereinheit 320 getrennten Datenbankserver untergebracht ist. Handelt es sich bei der Datenbank 330 um eine externe Datenbank, kann der Datenbankserver ein physischer Datenbankserver oder ein virtueller Datenbankserver sein. Der Datenbankserver kann vor Ort, z. B. in einem Rechenzentrum eines Krankenhauses, oder außerhalb des Hauses, z. B. in einem von einem Cloud-Computing-Dienstleister bereitgestellten Rechenzentrum, bereitgestellt werden.

Die Endoskop-Aufbereitungsmaschinen 301, 302, 303 und die Kamerasteuerungen 305, 306 übertragen wiederholt Statusinformationsdaten über das Netzwerk 350. Die Statusinformationsdaten werden von der Steuereinheit 320 empfangen.

Die von den Endoskop-Aufbereitungsmaschinen 301, 302, 303 bereitgestellten Statusdaten umfassen sowohl Statusdaten, die sich auf die jeweilige Endoskop-Aufbereitungsmaschine selbst beziehen, als auch Statusdaten, die sich auf einzelne Endoskope beziehen, die derzeit in der jeweiligen Endoskop-Aufbereitungsmaschine aufbereitet werden.

Eine nicht erschöpfende Liste von Statusinformationsdaten in Bezug auf eine Wiederaufbereitungsmaschine kann Folgendes umfassen:
- Zeit, die benötigt wird, um eine bestimmte Menge an Frischwasser zu entnehmen,
- Zeit, die benötigt wird, um einen Aufbereitungslösung auf eine bestimmte Temperatur zu erhitzen,
- Dosiermenge des Reinigungsmittels,
- Dosiermenge des Desinfektionsmittels.

Eine nicht erschöpfende Liste von Statusinformationsdaten in Bezug auf ein Endoskop kann Folgendes umfassen:
- Daten zur Identifizierung des Endoskops,
- Typ des Endoskops,
- Maximaler Druckabfall während des Durchlässigkeitstests der Kanäle,
- Maximale Leckagerate bei der Dichtheitsprüfung.

Die von den Kamerasteuerungen 305, 306 bereitgestellten Statusinformationsdaten umfassen Statusinformationsdaten in Bezug auf Endoskope, die derzeit mit der jeweiligen Kamerasteuerung verbunden sind. Für die vorliegende Offenlegung gehören zu den relevantesten Statusinformationsdaten Daten, die zeigen, dass ein entsprechendes Endoskop zusammen mit einer Kamerasteuerung 305, 306 verwendet wurde und daher vor der Verwendung bei einem anderen Patienten wieder aufbereitet werden muss. Zu den von den Kamerasteuerungen 305, 306 bereitgestellten Statusinformationen können auch Statusinformationen gehören, die sich direkt auf die jeweilige Kamerasteuerung beziehen.

Die Steuereinheit 320 speichert den Status der empfangenen Informationsdaten in der Datenbank 330, so dass die Statusinformationsdaten für einen späteren Abruf verfügbar sind.

Treten während eines Aufbereitungsvorgangs Fehler auf, liefert das jeweilige Endoskop-Aufbereitungsgerät zusätzliche Statusinformationen, die einen Fehlercode enthalten, der den Fehler identifiziert. Fehler können sich auf ein bestimmtes Endoskop oder eine bestimmte Endoskop-Aufbereitungsgerät beziehen.

Eine nicht erschöpfende Liste von endoskop-bezogenen Fehlern kann Folgendes umfassen:
- Unvollständiger Anschluss des Adapters,
- Nicht bestandene Dichtheitsprüfung,
- Nicht bestandener Durchgängigkeitstest.

Eine nicht erschöpfende Liste von maschinenbedingten Fehlern kann Folgendes umfassen:
- Die Zieltemperatur wird nicht erreicht,
- Die vorgegebene Wassermenge kann nicht entnommen werden,
- Versagen der Entwässerung,
- Chemikalienbehälter nicht angeschlossen,
- Unzureichende Chemikalienreserve,
- Versagen der Korbkupplung,
- Ausfall der Tür,
- Fehlerhafte Identifizierung des Endoskops.

Wenn die Steuereinheit 320 Statusinformationsdaten empfängt, die einen Fehlerzustand anzeigen, werden Fehlerinformationsdaten in Form einer Fehlermeldung einem Benutzer oder Bediener des Systems 300 über die Benutzerschnittstelle 340 zur Verfügung gestellt. Die Fehlerinformationsdaten enthalten Identifikationsdaten zur Identifizierung des medizinischen Geräts, bei dem der Fehlerzustand aufgetreten ist, wie z. B. einen Maschinenidentifizierungscode oder einen Endoskopidentifizierungscode. Die Fehlerinformationsdaten umfassen ferner Statusinformationsdaten des jeweiligen medizinischen Geräts.

Da der Fehlercode für verschiedene Typen von Endoskop-Aufbereitungsmaschinen spezifisch ist, wäre es für den Benutzer oder Bediener schwierig, die Bedeutung des Fehlers zu beurteilen, ohne z.B. die individuelle Maschinendokumentation heranzuziehen. Die Steuereinheit 320 ordnet daher jedem Fehlerzustand mindestens eine globale Fehlerkategorie aus einer Liste globaler Fehlerkategorien zu und nimmt die zugeordneten globalen Fehlerkategorien in die Fehlerinformationsdaten auf.

Die globalen Fehlerkategorien sind unabhängig von einem bestimmten Endoskop-Aufbereitungsgerät und können die Bedeutung eines jeweiligen Fehlers anzeigen. Eine nicht erschöpfende Liste globaler Fehlerkategorien, die die Wichtigkeit des Fehlers anzeigen, kann Folgendes umfassen:
- Sehr hohe Bedeutung (Wiederaufbereitungsverfahren unterbrochen, manueller Eingriff erforderlich),
- Hohe Bedeutung (Aufbereitungsverfahren abgeschlossen, aber für ein oder mehrere Endoskope ungültig),
- Mittlere Bedeutung (Wiederaufbereitungsverfahren abgeschlossen, aber Betriebsparameter nahe an den regulatorischen Grenzwerten),
- Geringe Bedeutung (Wiederaufbereitungsverfahren erfolgreich, aber manueller Eingriff vor dem nächsten Verfahren oder in naher Zukunft erforderlich).

Beispiele für Fehler in der Fehlerkategorie mit sehr hoher Wichtigkeit können sein: Versagen der Tür (z. B. unvollständiges Schließen einer automatischen Tür), Versagen der Korbkupplung, unvollständiger Anschluss des Adapters, fehlgeschlagener Leckagetest, Nichterreichen der Zieltemperatur, Nichterreichen der vorgegebenen Wassermenge, Versagen der Drainage, nicht angeschlossener Chemikalienbehälter und/oder unzureichender Chemikalienvorrat. Beispiele für Fehler in der Kategorie der Fehler von hoher Wichtigkeit können sein: fehlgeschlagener Durchgängigkeitstest und/oder fehlgeschlagene Identifizierung des Endoskops. Die Fehlerkategorie mit sehr hoher Wichtigkeit und die Fehlerkategorie mit hoher Wichtigkeit können in eine einzige Fehlerkategorie wie eine Fehlerkategorie mit hoher Wichtigkeit integriert werden. Fehler der Fehlerkategorie mit hoher Bedeutung können als "rote Fehler" bezeichnet werden.

Beispiele für Fehler der mittleren Fehlerkategorie können sein: niedriger Druck oder niedrige Temperatur der Frischwasserzufuhr, was zu einer verlängerten Dosier- oder Aufheizzeit führt, und/oder langsamer Druckaufbau im Durchgängigkeitstest aufgrund von Schaumbildung. Fehler der mittleren Fehlerkategorie können als "gelbe Fehler" bezeichnet werden.

Beispiele für Fehler der Kategorie "Fehler von geringer Bedeutung" können sein: Vorabinformationen über eine UV-Sterilisationseinheit, die sich dem Ende ihrer Lebensdauer nähert, den Inhalt eines Chemikalienbehälters, der sich dem Ende seiner Haltbarkeit nähert, und/oder bevorstehende geplante Wartungsarbeiten wie die Entnahme von Bioproben aus einer Aufbereitungsmaschine. Fehler der Kategorie "Fehler von geringer Bedeutung" können auch als "Vorabmeldungen" bezeichnet werden.

Die Liste der globalen Fehlerkategorien kann auch Fehlerkategorien umfassen, die anzeigen, ob ein Fehler auf einen menschlichen Fehler zurückzuführen ist. Entsprechende Fehlerkategorien können sein:
- Maschinenfehler,
- Menschliches Versagen.

Für die Zuordnung der einen oder mehreren globalen Fehlerkategorien zu einem bestimmten Fehlerzustand kann die Steuereinheit 320 auf Informationen der Datenbank 330 zugreifen. Für die Zuordnung von globalen Fehlerkategorien zu bestimmten Fehlerzuständen können verschiedene Ansätze angewendet werden.

Bei einem deterministischen Ansatz kann jedem spezifischen Fehlercode manuell eine globale Fehlerkategorie zugewiesen werden. Eine Liste von Fehlercodes und zugeordneten Fehlerkategorien kann dann in der Datenbank 330 in Form einer Nachschlagetabelle (LUT) gespeichert werden. Nach dem Empfang eines Fehlercodes kann die Steuereinheit 320 auf die LUT in der Datenbank 330 zugreifen und die entsprechende Fehlerkategorie aus der LUT lesen. Ein solcher Ansatz eignet sich besonders für die Zuweisung globaler Fehlerkategorien, die das Auftreten eines bestimmten Fehlerzustands anzeigen.

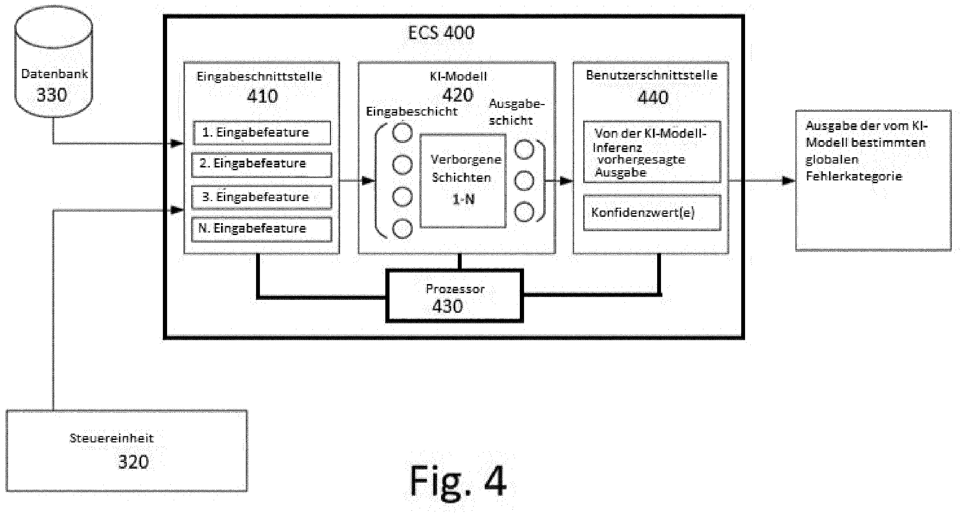

Bei einem nicht-deterministischen Ansatz, der Technologien des maschinellen Lernens verwendet, kann einem Fehlerzustand durch Technologien des maschinellen Lernens eine globale Fehlerkategorie zugewiesen werden. Ein solcher Ansatz eignet sich zum Beispiel für die Zuweisung globaler Fehlerkategorien, die anzeigen, ob ein Fehlerzustand auf menschliches Versagen zurückzuführen ist. Dies wird weiter unten mit Bezug auf Fig. 4 näher erläutert.

Fig. 4 zeigt ein schematisches Diagramm eines beispielhaften computergestützten Fehlerkategorisierungssystems (ECS) 400, das so eingerichtet ist, dass es auf der Grundlage von Statusinformationsdaten, die von einem medizinischen Gerät bereitgestellt werden, einem bestimmten Fehlerzustand eine globale Fehlerkategorie zuweist. In verschiedenen Ausführungsformen umfasst das ECS 400 eine Eingabeschnittstelle 410, über die Statusinformationsdaten, die für einen Fehlerzustand spezifisch sind, als 1. bis N. Eingabemerkmale für ein Modell der künstlichen Intelligenz (KI) 420 bereitgestellt werden, einen Prozessor 430, der eine Inferenzoperation durchführt, bei der die Statusinformationsdaten auf das Kl-Modell 420 angewendet werden, um die globale Fehlerkategorie zu erzeugen, und eine Benutzerschnittstelle (UI) 440, über die die globale Fehlerkategorie einem Benutzer, z. B. einem Bediener des Systems 300, mitgeteilt wird. Die Ul 440 kann in die Benutzeroberfläche 340 des Systems 300 integriert sein. In einigen Ausführungsformen kann das ECS 400 ein integraler Bestandteil der Steuereinheit 320 sein.

In einigen Ausführungsformen kann die Eingabeschnittstelle 410 eine direkte Datenverbindung zwischen dem ECS 400 und der Steuereinheit 320 sein. Beispielsweise kann die Eingabeschnittstelle 410 Informationsdaten direkt an das ECS 400 übermitteln, wenn ein Fehlerzustand bei einem angeschlossenen medizinischen Gerät auftritt. Zusätzlich oder alternativ kann die Eingabeschnittstelle 410 eine klassische Benutzerschnittstelle sein, die die Interaktion zwischen einem Benutzer und dem ECS 400 erleichtert. Zum Beispiel kann die Eingabeschnittstelle 410 eine Benutzerschnittstelle sein, über die der Benutzer manuell eine globale Fehlerkategorie eingeben kann, die einem Fehlerzustand während einer Lernphase des ECS 400 zugeordnet werden soll. Zusätzlich oder alternativ kann die Eingabeschnittstelle 410 dem ECS 400 den Zugriff auf die Datenbank 330 ermöglichen, um auf historische Statusinformationen desselben oder eines anderen medizinischen Geräts zuzugreifen, die in der Datenbank 330 gespeichert sind. In jedem dieser Fälle kann die Eingabeschnittstelle 410 so eingerichtet sein, dass sie aktuelle und historische Statusinformationen in Verbindung mit einem bestimmten medizinischen Gerät zu oder vor einem Zeitpunkt erfasst, zu dem das ECS 400 verwendet wird, um einem bestimmten Fehlerzustand eine globale Fehlerkategorie zuzuordnen.

Auf der Grundlage der Statusinformationsdaten führt der Prozessor 430 eine Inferenzoperation unter Verwendung des KI-Modells 420 durch, um eine globale Fehlerkategorie zu erzeugen, die dem spezifischen Fehlerzustand zugeordnet wird. Beispielsweise kann die Eingabeschnittstelle 410 die Statusinformationsdaten in eine Eingabeschicht des KI-Modells 420 liefern, die diese Eingabemerkmale durch das Kl-Modell an eine Ausgabeschicht weiterleitet. Das KI-Modell 420 kann ein Computersystem in die Lage versetzen, Aufgaben auszuführen, ohne explizit programmiert zu werden, indem es auf der Grundlage von Mustern, die bei der Analyse von Daten gefunden werden, Schlussfolgerungen zieht. Ein KI-Modell befasst sich mit der Untersuchung und Konstruktion von Algorithmen (z. B. Algorithmen des maschinellen Lernens), die aus vorhandenen Daten lernen und Vorhersagen über neue Daten treffen können. Solche Algorithmen bauen ein Kl-Modell anhand von Trainingsdaten auf, um datengestützte Vorhersagen oder Entscheidungen in Form von Ergebnissen oder Bewertungen zu treffen.

Es gibt zwei gängige Verfahren für maschinelles Lernen (ML): überwachtes ML und unüberwachtes ML. Überwachtes ML nutzt Vorwissen (z. B. Beispiele, die Eingaben mit Ausgaben oder Ergebnissen korrelieren), um die Beziehungen zwischen den Eingaben und den Ausgaben zu lernen. Das Ziel des überwachten ML ist es, eine Funktion zu erlernen, die bei gegebenen Trainingsdaten die Beziehung zwischen den Trainingsinputs und -outputs am besten annähert, so dass das ML-Modell dieselben Beziehungen implementieren kann, wenn ihm Inputs gegeben werden, um die entsprechenden Outputs zu erzeugen. Unüberwachtes ML ist das Training eines ML-Algorithmus unter Verwendung von Informationen, die weder klassifiziert noch etikettiert sind, und die es dem Algorithmus erlauben, auf diese Informationen ohne Anleitung zu reagieren. Unüberwachtes ML ist bei der explorativen Analyse nützlich, da es automatisch Strukturen in den Daten erkennen kann.

Übliche Aufgaben für überwachtes ML sind Klassifizierungsprobleme und Regressionsprobleme. Klassifizierungsprobleme, die auch als Kategorisierungsprobleme bezeichnet werden, zielen auf die Klassifizierung von Objekten in eine von mehreren Kategorien ab (z. B. Ist dieses Objekt ein Apfel oder eine Orange?). Regressionsalgorithmen zielen auf die Quantifizierung einiger Elemente ab (z. B. durch die Bereitstellung einer Punktzahl für den Wert einer Eingabe). Einige Beispiele für häufig verwendete überwachte ML-Algorithmen sind logistische Regression (LR), Naive-Bayes, Random Forest (RF), neuronale Netze (NN), Deep Neural Networks (DNN), Matrixfaktorisierung und Support Vector Machines (SVM).

Zu den üblichen Aufgaben des unüberwachten ML gehören Clustering, Repräsentationslernen und Dichteschätzung. Einige Beispiele für häufig verwendete unüberwachte ML-Algorithmen sind K-Means-Clustering, Hauptkomponentenanalyse und Autoencoder.

Eine andere Art des maschinellen Lernens ist das föderierte Lernen (auch bekannt als kollaboratives Lernen), bei dem ein Algorithmus auf mehreren dezentralen Geräten mit lokalen Daten trainiert wird, ohne dass die Daten ausgetauscht werden. Dieser Ansatz steht im Gegensatz zu herkömmlichen zentralisierten maschinellen Lerntechniken, bei denen alle lokalen Datensätze auf einen Server hochgeladen werden, sowie zu klassischeren dezentralen Ansätzen, bei denen häufig davon ausgegangen wird, dass die lokalen Datenproben identisch verteilt sind. Föderiertes Lernen ermöglicht es mehreren Akteuren, ein gemeinsames, robustes maschinelles Lernmodell zu erstellen, ohne Daten gemeinsam zu nutzen, wodurch kritische Fragen wie Datenschutz, Datensicherheit, Datenzugriffsrechte und Zugang zu heterogenen Daten gelöst werden können.

In einigen Beispielen kann das KI-Modell 420 kontinuierlich oder periodisch trainiert werden, bevor der Prozessor 430 die Inferenzoperation durchführt. Während der Inferenzoperation können dann die dem KI-Modell 420 bereitgestellten Statusinformationsdaten von einer Eingabeschicht über eine oder mehrere verborgene Schichten und schließlich zu einer Ausgabeschicht propagiert werden, die der globalen Fehlerkategorie entspricht, die einem bestimmten Fehlerzustand zuzuordnen ist. Wenn beispielsweise eine Endoskop-Aufbereitungsmaschine 301, 302, 303 einen Fehler meldet, weil eine Verbindungskraft, die erforderlich ist, um einen Endoskopkorb vollständig mit einem Spülsystemanschluss zu verbinden, einen Schwellenwert überschreitet, kann das KI-Modell 420 historische Daten wie das letzte Mal, als eine Adapterplatte des Korbs gewechselt wurde, Verbindungskräfte, die während früherer Kopplungsvorgänge gemessen wurden, und Ähnliches verwenden, um zu bestimmen, ob der Fehlerzustand das Ergebnis eines menschlichen Fehlers ist (z. B. falsche Platzierung der Adapterplatte) oder eines Maschinenfehlers (z. B. Degradation von Dichtungen o. ä.) ist, und eine entsprechende globale Fehlerkategorie als Ausgabe bereitzustellen. Zusammen mit der ermittelten globalen Fehlerkategorie kann das KI-Modell 420 ein Konfidenzniveau der Bestimmung liefern, das von der Trainingsqualität des Kl-Modells 420 abhängt. Wenn der bereitgestellte Konfidenzgrad einen vorgegebenen Schwellenwert nicht erreicht, kann ein Benutzer oder Bediener aufgefordert werden, die Statusdaten manuell zu analysieren, um zu bestimmen, ob der Fehlerzustand auf einen menschlichen oder maschinellen Fehler zurückzuführen ist, und das Ergebnis der manuellen Bestimmung über die Eingabeschnittstelle 410 an das KI-Modell 420 zu übermitteln. Diese Eingaben können dann verwendet werden, um das Kl-Modell 420 weiter zu trainieren, so dass künftige Inferenzoperationen bessere Konfidenzniveaus liefern können.

Während und/oder nach der Inferenzoperation kann die globale Fehlerkategorie an die Steuereinheit 320 übermittelt werden, um sie in die Fehlerinformationsdaten aufzunehmen, die dem Benutzer oder Bediener zur Verfügung gestellt werden.

Die Steuereinheit 320 kann eine Kombination aus deterministischen und nicht-deterministischen Ansätzen wie oben beschrieben verwenden, um die eine oder mehrere globale Fehlerkategorien einem bestimmten Fehlerzustand zuzuordnen.

Neben der globalen Fehlerkategorie können die Fehlerinformationsdaten auch Daten enthalten, die den Bediener auffordern, ein Fehlerbehandlungsverfahren in Abhängigkeit von der einen oder mehreren Fehlerkategorien durchzuführen. Fehlerbehandlungsverfahren können eines oder mehrere der folgenden Verfahren umfassen: Aufzeichnung vordefinierter oder editierbarer Protokolleinträge in den Protokollen des medizinischen Geräts, bei dem der Fehlerzustand aufgetreten ist; Kennzeichnung aller oder einiger Endoskope, die an einem betroffenen Aufbereitungsverfahren beteiligt sind, als fällig für eine erneute Aufbereitung; und Kennzeichnung eines Endoskop-Aufbereitungsgeräts, das von dem Fehlerzustand betroffen ist, als fällig für eine Wartung vor Beginn des nächsten Aufbereitungsverfahrens.

Die Fehlerinformationsdaten können darüber hinaus Informationen zur Fehlerbehebung umfassen, die für das medizinische Gerät spezifisch sind, bei dem der Fehlerzustand aufgetreten ist. Wenn beispielsweise ein Endoskop-Aufbereitungsgerät 301, 302, 303 einen Fehler meldet, weil eine Verbindungskraft, die erforderlich ist, um einen Endoskopkorb vollständig mit einem Spülsystemanschluss zu verbinden, einen Schwellenwert überschreitet, können die von der Steuereinheit 320 bereitgestellten Fehlerinformationsdaten eine Kopie oder einen Link zu einem Abschnitt eines Benutzerhandbuchs des betroffenen Endoskop-Aufbereitungsgeräts enthalten, der sich mit der Überprüfung oder Korrektur der Funktion der Korbkupplung befasst.

Unabhängig von der Ausgabe der Fehlerinformationsdaten über die Benutzerschnittstelle 340 empfängt die Steuereinheit 320 weiterhin Statusinformationsdaten von dem einen oder den mehreren medizinischen Geräten. Wenn für ein Gerät, das zuvor einen Fehlerzustand gemeldet hat, die aktuellen Statusinformationen keinen Fehlerzustand mehr anzeigen, wird die Ausgabe der Fehlerinformationen beendet, d.h. die Fehlermeldung wird von der Benutzerschnittstelle 340 gelöscht.

Neben der Ausgabe von Fehlerinformationsdaten, die sich auf einzelne Fehlerzustände beziehen, kann die Steuereinheit 320 auch statistische Daten zu den Fehlerzuständen berechnen. Solche statistischen Daten können die Anzahl oder die Häufigkeit bestimmter Fehlerzustände oder Fehler einer bestimmten globalen Fehlerkategorie umfassen. Beispielsweise kann eine Anzahl von Fehlerzuständen, die innerhalb der letzten Woche, des letzten Monats, des letzten Quartals oder eines anderen auswählbaren Zeitraums als "sehr wichtig" eingestuft wurden, als textliche oder grafische Information über die Benutzerschnittstelle 340 bereitgestellt oder in einem globalen Fehlerprotokoll in der Datenbank 330 gespeichert werden. In ähnlicher Weise kann eine kumulierte oder durchschnittliche Ausfallzeit in Bezug auf einen bestimmten Fehlerzustand oder eine bestimmte globale Fehlerkategorie berechnet, angezeigt und gespeichert werden. Zum Beispiel kann eine kumulierte Ausfallzeit von Endoskop-Aufbereitungsmaschinen aufgrund von menschlichem Versagen berechnet und angezeigt werden. Diese Informationen können dann vom Management verwendet werden, um die Notwendigkeit bestimmter Bedienerschulungen zu bestimmen, damit die durch menschliches Versagen verursachten Ausfallzeiten reduziert werden können.

Weiterhin ist es möglich, eine Rangfolge bestimmter Fehlerzustände oder globaler Fehlerkategorien nach ihrer Häufigkeit und/oder der durch die jeweiligen Fehlerzustände oder globalen Fehlerkategorien verursachten Ausfallzeit zu berechnen, anzuzeigen und zu speichern. Solche Informationen können vom Management zur Fokussierung von Verbesserungsmaßnahmen genutzt werden.

Die Steuereinheit 320 kann ferner so eingerichtet sein, dass sie Statusinformationsdaten von medizinischen Geräten im System 300 über ein globales Kommunikationsnetz 500, z. B. das Internet, an ein externes Datenzentrum 600 übermittelt. Zwischen der Steuereinheit 320 und dem Kommunikationsnetz 500 kann eine Firewall (nicht dargestellt) oder ein anderer Schutz gegen bösartige Kommunikationsinhalte vorgesehen sein.

Im externen Rechenzentrum 600 kann ein rechnergestütztes Fehleranalyse- und Vorhersagesystem EAPS implementiert werden. Der grundsätzliche Aufbau und die Funktionsweise des EAPS kann dem oben beschriebenen Aufbau und der Funktionsweise des ECS 400 sehr ähnlich sein, so dass hier der Kürze halber auf eine detaillierte Beschreibung verzichtet wird. Das EAPS verwendet vorzugsweise ML zur Erkennung von Mustern in Statusinformationsdaten, die einem Übergang eines medizinischen Geräts von einem regulären (fehlerfreien) Zustand in einen Fehlerzustand vorausgehen.

Die Statusinformationsdaten und der dem Fehlerzustand entsprechende Fehlercode werden dem EAPS als Trainingsdaten zur Verfügung gestellt, wobei die Statusinformationsdaten als Eingabe und der Fehlercode des aufgetretenen Fehlerzustands als Zielausgabe zur Verfügung gestellt werden. Durch wiederholtes Training "lernt" das EAPS, z.B. durch Anwendung von Mustererkennungsalgorithmen, Datenmuster in den Statusinformationsdaten zu erkennen, die den Übergang in den jeweiligen Fehlerzustand vorhersagen. Die beschriebene Lernmethode kann formal als "unüberwachtes Lernen" bezeichnet werden, da die Trainingsdaten keine von menschlichen Experten bereitgestellten Anmerkungen umfassen. Die durch Instanzen außerhalb des EAPS bereitgestellten Fehlercodes haben jedoch eine ähnliche Funktion wie die Anmerkungen menschlicher Experten, so dass die Lernmethode eher dem "überwachten Lernen" entspricht und entsprechende Algorithmen angewendet werden können. Das EAPS kann ein künstliches neuronales Netz oder eine Support-Vektor-Maschine verwenden.

Nach ausreichendem Training kann das EAPS dann verwendet werden, um ähnliche Datenmuster in den von anderen medizinischen Geräten empfangenen Statusinformationen zu erkennen, die in verschiedenen Einrichtungen installiert sein können. Auf der Grundlage der Erkennung kann das EAPS vorhersagen, dass ein bestimmtes medizinisches Gerät zu einem bestimmten Zeitpunkt in der Zukunft mit einer bestimmten Wahrscheinlichkeit auf einen bestimmten Fehlerzustand stoßen wird. Ein solches Vorhersageergebnis kann dann an das Kontrollsystem 320 zurückgegeben werden, das die vorhergesagten Fehlerinformationsdaten zusammenstellen und über die Benutzerschnittstelle 340 ausgeben kann.

Die Informationsdaten über den vorhergesagten Fehler können Identifikationsdaten umfassen, die das medizinische Gerät identifizieren, für das der Fehler vorhergesagt wird, Statusinformationsdaten, die von dem medizinischen Gerät empfangen werden, für das der Fehler vorhergesagt wird, und Daten zur Identifizierung des vorhergesagten Fehlers, die den vorhergesagten Fehlerzustand identifizieren. Die Daten zur Identifizierung des vorhergesagten Fehlers können einen Fehlercode für den vorhergesagten Fehlerzustand umfassen.

Ähnlich wie oben in Bezug auf Fehlermeldungen beschrieben, kann die Steuereinheit 320 den vorausgesagten Fehlerzuständen eine oder mehrere vorausgesagte Fehlerkategorien aus einer Liste vorausgesagter Fehlerkategorien zuordnen, um die vorausgesagten Fehlerinformationsdaten für einen Benutzer oder Bediener des Systems 300 leichter verständlich zu machen. Die Liste der vorausgesagten Fehlerkategorien kann vorausgesagte Fehlerkategorien enthalten, die unterschiedliche Schweregrade der vorausgesagten Fehlerzustände anzeigen. Die Liste der vorausgesagten Fehlerkategorien kann ferner vorausgesagte Fehlerkategorien enthalten, die anzeigen, ob ein vorausgesagter Fehlerzustand unbekannt ist oder nicht. Im Zusammenhang mit der vorliegenden Offenbarung ist unter einem "unbekannten vorhergesagten Fehlerzustand" ein vorhergesagter Fehlerzustand zu verstehen, dessen Art noch nicht vollständig analysiert ist.

Das EAPS kann so trainiert oder "verdrahtet" werden, dass es fehlerprognostische Datenmuster erkennt, die vollständig verstanden werden. Wenn beispielsweise die Zeitspanne, die zum Aufheizen eines Flüssigkeitsbehälters auf eine Zieltemperatur in einem bestimmten Endoskop-Aufbereitungsgerät benötigt wird, langsam ansteigt, kann das EAPS vorhersagen, dass das betreffende Endoskop-Aufbereitungsgerät in Zukunft in einen Fehlerzustand geraten wird, weil der Flüssigkeitsbehälter die Zieltemperatur nicht innerhalb eines zulässigen Zeitraums erreicht.

Bei bekannten vorhergesagten Fehlerzuständen können die Daten über den vorhergesagten Fehlerzustand auch Informationen über mögliche Fehlervermeidungsmaßnahmen umfassen.

Wenn jedoch eine neue Generation medizinischer Geräte auf den Markt kommt, sind möglicherweise nicht alle Fehlermechanismen vollständig analysiert worden. Hier kann der EAPS eingesetzt werden, um Datenmuster, die neue Fehlermechanismen anzeigen, frühzeitig zu erkennen, die dann zur weiteren Untersuchung solcher Fehlermechanismen genutzt werden können. Gleichzeitig kann das EAPS lernen, "unbekannte" Fehlerzustände vorherzusagen, so dass ein Benutzer des Systems 300 geeignete Gegenmaßnahmen ergreifen kann, um sich auf den erwarteten Ausfall des Geräts einzustellen.

Wenn beispielsweise ein oder mehrere Endoskope eines bestimmten Typs eine Dichtheitsprüfung vorzeitig, d. h. vor dem erwarteten Ende der Lebensdauer des Endoskops, nicht bestehen, kann der EAPS die Statusinformationsdaten dieser Endoskope in der Zeit vor dem Ausfall analysieren und Datenmuster erkennen, die den Ausfall vorhersagen.

Als rein fiktives Beispiel kann der EAPS erkennen, dass die jeweiligen Endoskope nach mehrmaliger Aufbereitung mit einer Aufbereitungschemikalie aus einer bestimmten Produktionscharge ausfallen, nicht aber nach der gleichen Anzahl von Aufbereitungen mit der gleichen Aufbereitungschemikalie aus einer anderen Produktionscharge. Der EAPS kann dann Statusinformationen aus derselben oder anderen Einrichtungen auswerten und die jeweiligen Steuereinheiten auffordern, einem Benutzer oder Bediener vorausgesagte Fehlerinformationsdaten zu präsentieren, wenn Endoskope des betroffenen Typs mit der Aufbereitungschemikalie aus der betroffenen Produktionscharge aufbereitet werden.

Als weiteres fiktives Beispiel kann der EAPS erkennen, dass bei einem bestimmten Typ von Endoskopen häufig ein Fehler auftritt, weil die Verbindungsschläuche von den Endoskopanschlüssen abrutschen, wenn das Endoskop in einem bestimmten Typ von Endoskop-Aufbereitungsgeräten oder in einer bestimmten Aufbereitungsposition innerhalb eines Endoskop-Aufbereitungsgeräts, wie Position 1, Position 2 oder dergleichen, aufbereitet wird. Der EAPS kann dann Statusinformationen derselben oder anderer Einrichtungen analysieren und die jeweiligen Steuereinheiten dazu veranlassen, einem Benutzer oder Bediener vorausgesagte Fehlerinformationsdaten zu präsentieren, wenn ein Endoskop des betroffenen Typs für die Aufbereitung in dem betroffenen Aufbereitungsgerätetyp und/oder an der betroffenen Aufbereitungsposition vorgesehen ist. In diesem speziellen Fall können die prognostizierten Fehlerdaten einen Hinweis darauf enthalten, dass beim Anschließen des Endoskops an die Anschlussschläuche besondere Vorsicht geboten ist.

## Patentansprüche

1. Verfahren zur Handhabung von Statusinformationen medizinischer Geräte, mit den Schritten:
Empfangen von Statusinformationsdaten von einem oder mehreren medizinischen Geräten, wobei die Statusinformationsdaten in einem für jedes medizinische Gerät spezifischen Datenformat empfangen werden;
Bestimmen, dass die Statusinformationsdaten einen Fehlerzustand des einen oder der mehreren medizinischen Geräte anzeigen;
Zuordnen einer oder mehrerer globaler Fehlerkategorien aus einer vorbestimmten Liste globaler Fehlerkategorien zu jedem Fehlerzustand, wobei die globalen Fehlerkategorien nicht spezifisch für ein bestimmtes medizinisches Gerät sind; und
Ausgeben von Fehlerinformationsdaten, die umfassen
- Identifikationsdaten, die ein spezifisches medizinisches Gerät identifizieren,
- Statusinformationsdaten, die von dem spezifischen medizinischen Gerät empfangen wurden, und
- die eine oder mehrere globalen Fehlerkategorien, die dem erkannten Fehlerstatus zugeordnet sind.

2. Verfahren nach Anspruch 1, wobei die Liste der Fehlerkategorien Fehlerkategorien enthält, die unterschiedliche Schweregrade der jeweiligen Fehlerzustände anzeigen.

3. Verfahren nach Anspruch 2, wobei die Liste der Fehlerkategorien eine oder mehrere der folgenden Kategorien enthält:
- eine erste Fehlerkategorie, die einen Fehler mit hohem Schweregrad anzeigt, und
- eine zweite Fehlerkategorie, die einen Fehler mittleren Schweregrads anzeigt.

4. Verfahren nach Anspruch 3, wobei die Liste der Fehlerkategorien ferner eine dritte Fehlerkategorie enthält, die einen Fehler geringen Schweregrads anzeigt.

5. Verfahren nach einem der Ansprüche 1 bis 4, wobei die Liste der Fehlerkategorien Fehlerkategorien enthält, die anzeigen, ob der Fehlerzustand durch menschliches Versagen verursacht wurde oder nicht.

6. Verfahren nach einem der vorstehenden Ansprüche, wobei die Fehlerinformationsdaten Daten enthalten, die einen Bediener auffordern, eine Fehlerbehandlungsprozedur durchzuführen, wobei die Fehlerbehandlungsprozedur von der einen oder den mehreren Fehlerkategorien abhängt, die dem Fehlerzustand zugeordnet sind.

7. Verfahren nach Anspruch 6, wobei die Fehlerinformationsdaten ferner Fehlerbehebungsinformationen enthalten, die spezifisch für das von dem Fehlerzustand betroffene medizinische Gerät sind.

8. Verfahren nach einem der obigen Ansprüche, das ferner umfasst wiederholtes Empfangen von Statusinformationsdaten von dem einen oder den mehreren medizinischen Geräten, und
Beenden der Ausgabe der Fehlerinformationsdaten, wenn die Statusinformation anzeigt, dass der Fehlerzustand behoben wurde.

9. Verfahren nach einem der obigen Ansprüche, das ferner umfasst:
Bereitstellen von statistischen Informationen einschließlich einer oder mehrerer der folgenden Informationen
- eine Anzahl oder Häufigkeit bestimmter Fehlerzustände oder Fehler einer bestimmten Fehlerkategorie innerhalb einer vorbestimmten oder wählbaren Zeitspanne,
- einer durchschnittlichen oder kumulierten Ausfallzeit, die durch bestimmte Fehlerzustände oder Fehler einer bestimmten Fehlerkategorie verursacht wird, oder
- eine Rangliste der häufigsten Fehlerzustände oder Fehlerkategorien.

10. Verfahren zur Behandlung von Statusinformationen medizinischer Geräte, mit den Schritten:
wiederholtes Empfangen von Statusinformationsdaten von einem oder mehreren medizinischen Geräten,
Speichern der empfangenen Statusinformationsdaten in einer Datenbank, Bestimmen, ob die Statusinformationsdaten einen Fehlerzustand eines ersten des einen oder der mehreren medizinischen Geräte anzeigen; und
nach der Bestimmung, dass die Statusinformationsdaten einen Fehlerzustand des ersten des einen oder der mehreren medizinischen Geräte anzeigen:
- Analysieren der gespeicherten Statusinformationsdaten auf erste Datenmuster, die den Fehlerzustand vorhersagen;
- Durchsuchen gespeicherter Statusinformationsdaten, die von mindestens einem zweiten des einen oder der mehreren medizinischen Geräte, das nicht das erste ist, empfangen wurden, nach zweiten Datenmustern, die den ersten Datenmustern ähnlich sind;
- Bestimmen, basierend auf einer Ähnlichkeit zwischen den zweiten Datenmustern und den ersten Datenmustern, einer Wahrscheinlichkeit, dass das zweite des einen oder der mehreren medizinischen Geräte innerhalb einer gegebenen Zeitspanne in einen Fehlerzustand geraten wird; und
- wenn die Wahrscheinlichkeit einen Schwellenwert überschreitet, Ausgeben von vorhergesagten Fehlerinformationsdaten, die umfassen
• Identifikationsdaten, die das zweite medizinische Gerät identifizieren,
• Statusinformationsdaten, die von dem zweiten medizinischen Gerät empfangen werden, und
• Daten zur Identifizierung des vorhergesagten Fehlers, die den vorhergesagten Fehlerzustand identifizieren.

11. Verfahren nach Anspruch 10, das ferner Folgendes umfasst Zuordnen mindestens einer vorhergesagten Fehlerkategorie aus einer vorbestimmten Liste von vorhergesagten Fehlerkategorien zu jedem vorhergesagten Fehlerzustand; und Ausgeben der zugewiesenen vorhergesagten Fehlerkategorie als Teil der vorhergesagten Fehleridentifikationsdaten.

12. Verfahren nach Anspruch 11, wobei die Liste der vorhergesagten Fehlerkategorien vorhergesagte Fehlerkategorien umfasst, die unterschiedliche Schweregrade der jeweiligen vorhergesagten Fehlerzustände anzeigen.

13. Verfahren nach Anspruch 11 oder 12, wobei die Liste der vorhergesagten Fehlerkategorien mindestens eine vorhergesagte Fehlerkategorie umfasst, die anzeigt, dass der vorhergesagte Fehlerzustand unbekannt ist.

14. Verfahren nach einem der Ansprüche 10 bis 13, wobei mindestens einer der Schritte Analyse, Suche und Bestimmung einen oder mehrere Mustererkennungsalgorithmen verwendet.

15. Verfahren nach einem der Ansprüche 10 bis 14, wobei mindestens einer der Schritte Analyse, Suche und Bestimmung unter Verwendung eines künstlichen neuronalen Netzes oder einer Support-Vektor-Maschine durchgeführt wird.

16. Ein System, umfassend
- mindestens eine Maschine zur Wiederaufbereitung von Endoskopen
- eine Datenbank, und
- eine Steuereinheit,
wobei die Steuereinheit so eingerichtet ist, dass sie das Verfahren nach einem der obigen Ansprüche durchführt.
